# EUROPÄISCHE PATENTSCHRIFT

(11) **EP 2 858 694 B1**
(45) Veröffentlichungstag und Bekanntmachung des Hinweises auf die Patenterteilung: **24.05.2017**
(21) Anmeldenummer: 13726118.6
(22) Anmeldetag: 31.05.2013
(51) Int. Cl.: A61M 1/16, A61M 1/34

(54) **VORRICHTUNG ZUR EXTRAKORPORALEN BLUTBEHANDLUNG**
APPARATUS FOR EXTRACORPOREAL BLOOD TREATMENT
DISPOSITIF POUR LE TRAITEMENT DU SANG EXTRACORPOREL

(30) Priorität: 06.06.2012 DE 102012011196; 06.06.2012 US 201261656058 P
(43) Veröffentlichungstag der Anmeldung: 15.04.2015
(73) Patentinhaber: Fresenius Medical Care Deutschland GmbH, 61352 Bad Homburg v.d.H. (DE); Fresenius Medical Care - DS, s.r.o., 160 00 Praha 6 (CZ)
(72) Erfinder: JIRKA, Tomás, 277 44 (CZ); JONAS, Jörg, Bogotá (CO); NIER, Volker, 61203 Reichelsheim (DE); WEHMEYER, Wolfgang, 72076 Tübingen (DE)
(74) Vertreter: Oppermann, Frank
(86) Internationale Anmeldenummer: PCT/EP2013/001608
(87) Internationale Veröffentlichungsnummer: WO 2013/182287

(56) Entgegenhaltungen:
- JP-A- 2004 049 493
- US-A- 4 234 428
- US-B1- 6 284 141
- US-B2- 6 821 421

## Beschreibung

Die Erfindung betrifft eine Vorrichtung zur extrakorporalen Blutbehandlung mit einem extrakorporalen Blutkreislauf, der eine erste Kammer eines durch eine semipermeable Membran in die erste Kammer und eine zweite Kammer unterteilten Dialysators oder Filters einschließt, und einem Dialysierflüssigkeitssystem, das die zweite Kammer des Dialysators oder Filters einschließt.

Zur Entfernung von harnpflichtigen Substanzen und zum Flüssigkeitsentzug werden beim chronischen Nierenversagen verschiedene Verfahren zur extrakorporalen Blutbehandlung eingesetzt (siehe z.B. JP 2004049493, und us6821421). Während bei der Hämodialyse (HD) der Stofftransport der kleinmolekularen Substanzen durch die semipermeable Membran im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe, durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren stellt die Hämodiafiltration (HDF) dar.

Bei der Hämodiafiltration wird ein Teil des durch die Membran des Dialysators oder Filters abgezogenen Ultrafiltrats durch eine sterile Substitutionsflüssigkeit (Substituat) ersetzt, die entweder stromauf oder stromab des Dialysators dem extrakorporalen Blutkreislauf zugeführt wird. Die Zufuhr von Substituat stromauf des Dialysators wird als Prädilution und die Zufuhr stromab des Dialysators als Postdilution bezeichnet.

Es sind Vorrichtungen zur Hämodiafiltration bekannt, bei denen die Dialysierflüssigkeit online aus Frischwasser und Konzentrat und das Substituat online aus der Dialysierflüssigkeit gewonnen werden. Bei den bekannten Hämodiafiltrationsvorrichtungen wird das Substituat im extrakorporalen Blutkreislauf von dem Dialysierflüssigkeitssystem der Maschine über eine Substituatleitung zugeführt. Hämodiafiltrationsvorrichtungen sind beispielsweise aus der EP 0 974 371 A3 und EP 1 595 560 A1 bekannt.

Bei der Dialyse können in einzelnen Fällen auf Grund von Elektrolytverschiebungen Komplikationen auftreten, die zu Kopfschmerzen oder psychomotorischen Störungen bei den Patienten führen. Diese Komplikationen sind als Dysäquilibrium-Syndrom bekannt.

Das Dysäquilibrium-Syndrom wird in der DE 32 23 051 A1 angesprochen. Die DE 32 23 051 A1 beschreibt eine Dialysevorrichtung, bei der die Zusammensetzung der Dialysierflüssigkeit während der Dialysebehandlung in Abhängigkeit von dem Elektrolytgehalt der Dialysierflüssigkeit stromauf und stromab des Dialysators geregelt wird. Der Elektrolytgehalt der Dialysierflüssigkeit wird mit stromauf und stromab des Dialysators angeordneten Sensoren gemessen.

Die JP 2004 049493 A beschreibt eine Vorrichtung zur extrakorporalen Blutbehandlung mit einem extrakorporalen Blutkreislauf und einem Flüssigkeitssystem, die den Betrieb als Hämodiafiltrationsvorrichtung erlaubt, bei der Dialysierflüssigkeit durch die Dialysierflüssigkeitskammer des Dialysators strömt und Ultrafiltrat aus der Dialysierflüssigkeitskammer des Dialysators abgezogen wird. Die Hämodiafiltrationsvorrichtung sieht einen initialen Behandlungsmodus vor, bei dem die Strömung von Dialysierflüssigkeit durch die Dialysierflüssigkeitskammer des Dialysators unterbrochen ist, so dass nur eine Ultrafiltration erfolgt. Der Übergang von dem initialen Behandlungsmodus auf den normalen Behandlungsmodus der Hämodiafiltration erfolgt automatisch. Ein initialer Behandlungsmodus mit einer Ultrafiltration ohne Dialysierflüssigkeitsfluss ist auch aus der US 4 234 428 A bekannt.

Aus der US 6 821 421 B2 ist eine Blutbehandlungsvorrichtung bekannt, die über einen Bypass im Dialysierflüssigkeitssystem verfügt, so dass die Blutbehandlung in einem Behandlungsmodus betrieben werden kann, in dem ein Teil der Dialysierflüssigkeit durch den Bypass strömt. Dadurch kann der Dialysierflüssigkeitsfluss in einer initialen Behandlungsphase reduziert werden. In einem Störfall kann die Blutbehandlung vollständig unterbrochen werden. Die US 6 821 421 B2 beschreibt eine Blutbehandlung, bei der zunächst eine niedrige Dialysierflüssigkeitsrate vorgegeben wird, die später schrittweise erhöht wird.

Der Erfindung liegt die Aufgabe zu Grunde, eine Vorrichtung zur extrakorporalen Blutbehandlung zu schaffen, die mit einem verringerten Risiko des Auftretens von Komplikationen für den Patienten betrieben werden kann.

Die Lösung dieser Aufgaben erfolgt erfindungsgemäß mit den Merkmalen der unabhängigen Patentansprüche. Die Gegenstände der abhängigen Ansprüche betreffen bevorzugte Ausführungsformen der Erfindung.

Es hat sich gezeigt, dass das Dysäquilibrium-Syndrom auf Elektrolytverschiebungen während der extrakorporalen Blutbehandlung zurückzuführen ist. Daher sollte eine übermäßige Reduktion von Elektrolyten, insbesondere Kalium, oder die übermäßige Reduktion von Harnstoff bei der extrakorporalen Blutbehandlung vermieden werden.

Die Erfindung beruht auf der Erkenntnis, dass eine besonders schnelle Entfernung von Elektrolyten wie Kalium oder von Harnstoff gerade während der ersten Phase einer Hämodiafiltration auftritt. Die Erfindung beruht somit auf dem Prinzip, die übermäßige Reduktion von Elektrolyten in der Anfangsphase der extrakorporalen Blutbehandlung zu vermeiden.

Zur Vermeidung einer übermäßigen Reduktion von Elektrolyten oder einer übermäßigen Reduktion von Harnstoff sieht die Erfindung einen initialen Behandlungsmodus vor, in dem die extrakorporale Blutbehandlungsvorrichtung innerhalb eines vorgegebenen Zeitintervalls nur als Hämofiltrationsvorrichtung betrieben wird. Während der Hämofiltration ist die Dialysierflüssigkeitsrate gleich Null, wobei über die Membran des Filters nur Plasmawasser entzogen wird. Die Austauschraten von Elektrolyten wie Kalium oder von Harnstoff reduzieren sich in der initialen Phase der extrakorporalen Blutbehandlung, da die Entfernung von Elektrolyten oder Harnstoff überwiegend diffusiv erfolgt und bei der Hämofiltration im Gegensatz zur Hämodialyse ein konvektiver Stoffaustausch erfolgt.

Die dem Patienten in der Anfangsphase der Blutbehandlung entzogene Menge an Flüssigkeit wird dem Patienten als Substituat wieder zugeführt. Der Anfangsphase der Blutbehandlung mit dem initialen Behandlungsmodus schließt sich die eigentliche Blutbehandlung mit den vom Arzt vorgegebenen Parametern an.

Eine alternative Ausführungsform sieht zur Vermeidung einer übermäßigen Reduktion von Elektrolyten oder Harnstoff in dem initialen Behandlungsmodus eine Förderung von Dialysierflüssigkeit mit einer ersten Dialysierflüssigkeitsrate vor, die kleiner als die zweite Dialysierflüssigkeitsrate ist, mit der die Dialysierflüssigkeit nach Ablauf des vorgegebenen Zeitintervalls gefördert wird. In diesem Zusammenhang werden unter einer ersten und zweiten Dialysierflüssigkeitsrate nicht zwingend konstante Flüssigkeitsraten verstanden. Insofern kann unter der ersten und zweiten Flüssigkeitsrate auch eine mittlere Flüssigkeitsrate verstanden werden. Für die Erfindung ist nur wesentlich, dass die Dialysierflüssigkeitsraten in dem initialen Behandlungsmodus kleiner als in einem dem initialen Behandlungsmodus nachfolgenden Behandlungsmodus sind. Vorzugsweise sollte die erste Dialysierflüssigkeitsrate sehr viel kleiner als die zweite Dialysierflüssigkeitsrate sein, insbesondere null sein.

Die erfindungsgemäße Vorrichtung zur extrakorporalen Blutbehandlung verfügt über Mittel zum Abziehen von Ultrafiltrat aus der Kammer des Dialysators oder Filters über die semipermeable Membran des Dialysators oder Filters mit einer vorgegebenen Ultrafiltratrate U_{f} in einem ersten Betriebsmodus und zum Fördern von Dialysierflüssigkeit mit einer vorgegebenen Dialysierflüssigkeitsrate Q_{d} durch eine der Kammern des Dialysators oder Filters in einem zweiten Betriebsmodus, wobei in dem ersten Betriebsmodus die Flüssigkeitsströmung in die Kammer des Dialysators oder Filters unterbrochen ist, so dass die Kammer nicht von Dialysierflüssigkeit durchströmt wird, oder nur eine kleine Dialysierflüssigkeitsrate eingestellt wird. In dem ersten Betriebsmodus kann die Blutbehandlungsvorrichtung also im Wesentlichen nur als Hämofiltrationsvorrichtung und in dem zweiten Betriebsmodus als Hämodiafiltrationsvorrichtung betrieben werden.

Für die Erfindung ist unerheblich, wie die Mittel zum Fördern von Dialysierflüssigkeit und Abziehen von Ultrafiltrat beschaffen sind. Daher werden unter diesen Mitteln sämtliche Mittel verstanden, die hierfür geeignet sind. Für die Erfindung ist auch unerheblich, wie Dialysierflüssigkeit und Substituat gewonnen und gefördert werden.

Die Steuer- und Recheneinheit der extrakorporalen Blutbehandlungsvorrichtung weist Mittel zur Vorgabe eines initialen Behandlungsmodus nur während der Anfangsphase der Blutbehandlung auf. Die Mittel zur Vorgabe des initialen Behandlungsmodus sind derart ausgebildet, dass innerhalb eines vorgegebenen Zeitintervalls Substituat dem extrakorporalen Blutkreislauf mit einer vorgegebenen Substituatrate Qₛ zugeführt wird, Dialysierflüssigkeit aber durch die Dialysierflüssigkeitskammer des Dialysators oder Filters nicht oder zumindest nur mit einer kleinen Dialysierflüssigkeitsrate gefördert wird, sondern im Wesentlichen nur über die Membran des Dialysators Ultrafiltrat abgezogen wird. Nach Ablauf des ersten Zeitintervalls schalten die Mittel zum Fördern von Dialysierflüssigkeit und Abziehen von Ultrafiltrat auf einen dem initialen Behandlungsmodus nachfolgenden Behandlungsmodus um, in dem die Mittel zum Fördern von Dialysierflüssigkeit und Abziehen von Ultrafiltrat in einem anderen Betriebsmodus betrieben werden, so dass Dialysierflüssigkeit erst jetzt oder Dialysierflüssigkeit nunmehr mit einer größeren Dialysierflüssigkeitsrate durch die zweite Kammer des Dialysators oder Filters gefördert wird.

Mit den Mitteln zur Vorgabe des initialen Behandlungsmodus wird bei der extrakorporalen Blutbehandlung sichergestellt, dass die Entfernungsrate von Elektrolyten, insbesondere von Kalium, oder von Harnstoff auf unschädliche Werte begrenzt wird, so dass die Gefahr des Auftretens der angesprochenen Komplikationen während der Blutbehandlung, insbesondere des Dysäquilibrium-Syndroms, gering ist.

Bei einer bevorzugten Ausführungsform sind die Mittel zur Vorgabe des initialen Behandlungsmodus derart ausgebildet, dass die Mittel zum Zuführen von Substituat und Abziehen von Substituat innerhalb des vorgegebenen Zeitintervalls, in dem die Blutbehandlungsvorrichtung im Wesentlichen nur als Hämofiltrationsvorrichtung betrieben wird, derart betrieben werden, dass die Substituatrate Qₛ innerhalb des Zeitintervalls erhöht wird. Vorzugsweise wird die Substituatrate innerhalb des Zeitintervalls kontinuierlich mit einer gleichbleibenden Steigerungsrate, vorzugsweise von einer Substituatrate Qₛ = 0 erhöht. Es ist aber auch möglich, dass die Substituatrate mit einer zunehmenden Steigerungsrate kontinuierlich erhöht wird. Beispielsweise kann die Substituatrate Qₛ exponentiell erhöht werden. Die Substituatrate wird innerhalb des Zeitintervalls von einem Anfangswert, der vorzugsweise Null ist, aber auch ein Wert größer als Null sein kann, auf einen vorgegebenen Zielwert erhöht.

Die Mittel zur Vorgabe des initialen Behandlungsmodus sind derart ausgebildet, dass nach Ablauf des vorgegebenen Zeitintervalls, in dem die Blutbehandlungsvorrichtung im Wesentlichen nur als Hämofiltrationsvorrichtung betrieben wird, die Mittel zum Fördern von Dialysierflüssigkeit und Abziehen von Ultrafiltrat innerhalb eines zweiten vorgegebenen Zeitintervalls, das sich unmittelbar an das erste Zeitintervall anschließt, derart betrieben werden, dass die Dialysierflüssigkeitsrate Qd auf einen vorgegebenen Wert erhöht wird. Die Dialysierflüssigkeitsrate Q_{d} kann innerhalb des vorgegebenen Zeitintervalls kontinuierlich mit einer gleichbleibenden Steigerungsrate oder kontinuierlich mit einer zunehmenden Steigerungsrate, beispielsweise exponentiell erhöht werden. Die Erhöhung der Dialysierflüssigkeitsrate erfolgt auf einen vorgegebenen Zielwert, mit dem die Blutbehandlung fortgesetzt werden soll. Die Dialysierflüssigkeitsrate kann in dem zweiten Zeitintervall auch bei der alternativen Ausführungsform kontinuierlich mit einer gleichbleibenden Steigerungsrate oder kontinuierlich mit einer zunehmenden Steigerungsrate erhöht werden. Die Erhöhung kann beispielsweise ausgehend von einer Dialysierflüssigkeitsrate erfolgen, die größer als die in dem ersten Zeitintervall eingestellte Dialysierflüssigkeitsrate ist.

Mit der Anpassung der Dialysierflüssigkeitsrate wird ein kontinuierlicher Übergang von der reinen Hämofiltration auf die Hämodiafiltration erreicht, was sich positiv auf das Befinden des Patienten auswirkt.

Eine weitere besonders bevorzugte Ausführungsform der Erfindung sieht eine Eingabeeinheit vor, auf der insbesondere der Patient selbst Eingaben machen kann, mit denen die Länge des ersten Zeitintervalls vorgegeben wird. Beispielsweise kann die Eingabeeinheit derart ausgebildet sein, dass der Patient aus zwei oder mehreren vorgegebenen Zeitintervallen unterschiedlicher Länge ein Zeitintervall auswählen kann. Es ist aber auch möglich, dass der Patient die Länge des Zeitintervalls kontinuierlich verlängern oder verkürzen kann, d.h. einstellen kann. Hierzu sind sämtliche Eingabemittel möglich. Die Eingabeeinheit gibt dem Patienten die Möglichkeit in Abhängigkeit von seinem momentanen Befinden die Länge des ersten Zeitintervalls vorzugeben. Beispielsweise kann der Patient durch Vorgabe eines langen Zeitintervalls von beispielsweise 60 Minuten, einen "weichen Übergang" vorgeben, so dass die Gefahr des Auftretens des Dysäquilibrium-Syndroms besonders gering ist. Der Patient kann aber auch durch Vorgabe eines kürzeren Zeitintervalls, beispielsweise von 30 Minuten, einen "härteren Übergang" vorgeben, so dass die extrakorporale Blutbehandlung während der Anfangsphase zwar effektiver als bei der Vorgabe des "weicheren Übergangs" ist, die Gefahr des Auftretens des Dysäquilibrium-Syndroms daher höher, aber immer noch gering ist.

Nach Ablauf des ersten und zweiten vorgegebenen Zeitintervalls kann die Hämodiafiltrationsbehandlung in bekannter Weise fortgesetzt werden, wobei die Dialysierflüssigkeits- und/oder Ultrafiltrat- und/oder Substituatrate in bekannter Weise unter Berücksichtigung unterschiedlicher Gesichtspunkte eingestellt wird. Für die Erfindung entscheidend ist nur, dass unabhängig von der Einstellung der Flüssigkeitsraten für die eigentliche Blutbehandlung in der Anfangsphase der Blutbehandlung ein initialer Behandlungsmodus automatisch vorgegeben wird, bei dem das Risiko des Auftretens des Dysäquilibrium-Syndroms stark verringert ist.

Im Folgenden wird ein Ausführungsbeispiel der Erfindung unter Bezugnahme auf die Zeichnungen im Einzelnen beschrieben.

Es zeigen:
- Fig. 1: die wesentlichen Komponenten der erfindungsgemäßen Vorrichtung zur extrakorporalen Blutbehandlung in stark vereinfachter schematischer Darstellung und
- Fig. 2: die Harnstoffkonzentration als Funktion der Behandlungszeit.

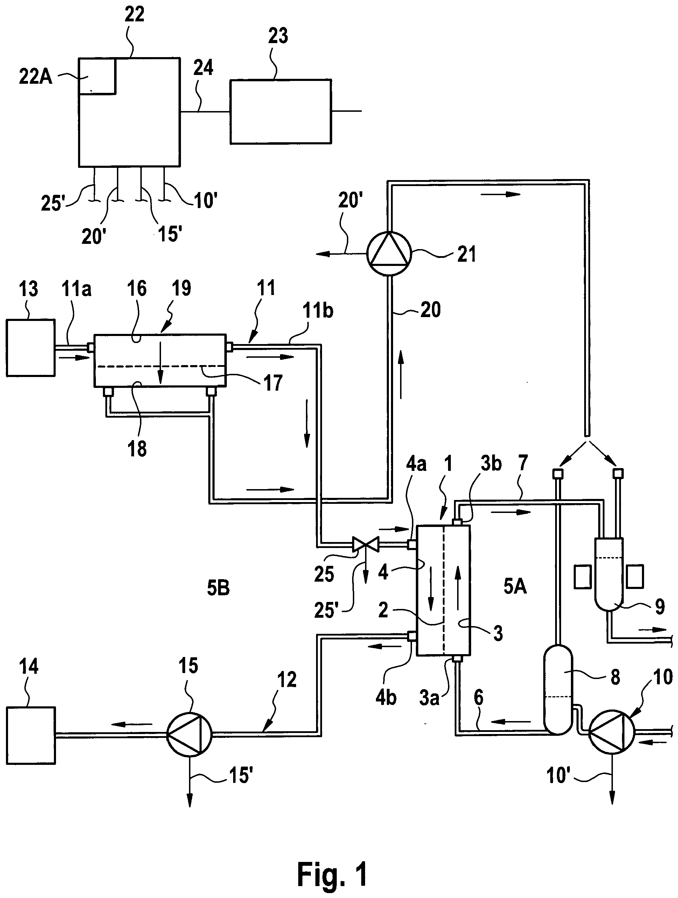

Fig. 1 zeigt eine vereinfachte schematische Darstellung der wesentlichen Komponenten einer Hämodiafiltrationsvorrichtung.

Die Hämodiafiltrationsvorrichtung weist einen Dialysator oder Filter 1 auf, der durch eine semipermeble Membran 2 in eine von Blut durchflossene erste Kammer 3 und eine von Dialysierflüssigkeit durchflossene zweite Kammer 4 getrennt ist. Wenn die Hämodiafiltrationsvorrichtung als Hämofiltrationsvorrichtung betrieben wird, fungiert der Dialysator als Filter.

Der extrakorporale Blutkreislauf 5A umfasst eine arterielle Blutleitung 6, die von dem Patienten zu dem Einlass 3a der Blutkammer 3 führt, und eine venöse Blutleitung 7, die von dem Auslass 3b der Blutkammer 3 des Dialysators 1 abgeht und zu dem Patienten führt. Zur Minimierung von Luftblasen sind in der arteriellen Blutleitung 6 eine arterielle Tropfkammer 8 und in der venösen Blutleitung 7 eine venöse Tropfkammer 9 vorgesehen. Das Blut des Patienten wird durch die Blutkammer 3 des Dialysators 1 mit einer arteriellen Blutpumpe 10 gefördert, die an der arteriellen Blutleitung 6 angeordnet ist.

Das Flüssigkeitssystem 5B umfasst eine Dialysierflüssigkeitszuführleitung 11, die zu dem Einlass 4a der Dialysierflüssigkeitskammer 4 führt, und eine Dialysierflüssigkeitsabführleitung 12, die von dem Auslass 4b der Dialysierflüssigkeitskammer 4 des Dialysators 1 abgeht. Über die Dialysierflüssigkeitszuführleitung 11 strömt frische Dialysierflüssigkeit aus einer Dialysierflüssigkeitsquelle 13 in die Dialysierflüssigkeitskammer 4, während verbrauchte Dialysierflüssigkeit aus der Dialysierflüssigkeitskammer 4 über die Dialysierflüssigkeitsabführleitung 12 zu einem Abfluss 14 abgeführt wird. Die Dialysierflüssigkeit wird mit einer an der Dialysierflüssigkeitsabführleitung 12 angeordneten Dialysierflüssigkeitspumpe 15 gefördert. In der Dialysierflüssigkeitszuführleitung 11 ist ein Absperrorgan 25 angeordnet, mit dem die Zufuhr von Dialysierflüssigkeit in die zweite Kammer des Dialysators und somit die Flüssigkeitsströmung durch die zweite Kammer des Dialysators unterbrochen werden kann.

Die in Hämodiafiltrationsvorrichtungen im Allgemeinen vorgesehene Bilanziereinrichtung zur Bilanzierung frischer gegen verbrauchte Dialysierflüssigkeit ist der besseren Übersichtlichkeit halber nicht dargestellt. Auch sind zusätzliche Einrichtungen, die beispielsweise Mittel zum Reinigen und Spülen des Systems und Bypass-Leitungen sowie weitere Absperrorgane umfassen können, nicht dargestellt.

Die Dialysierflüssigkeitszuführleitung 11 umfasst einen ersten Abschnitt 11a, der zu dem Einlass einer ersten Kammer 16 eines durch eine Membran 17 in die erste Kammer und eine zweite Kammer 18 unterteilten Sterilfilters 19 führt, und einen zweiten Abschnitt 11b, der von dem Auslass der ersten Kammer 16 des Filters 19 abgeht und zu dem Einlass 4a der Dialysierflüssigkeitskammer 4 führt.

Während der Blutbehandlung kann Dialysierflüssigkeit aus dem Dialysierflüssigkeitssystem 5B als Substituat über eine Substituatleitung 20, die von der zweiten Kammer 18 des Filters 19 abgeht, dem extrakorporalen Blutkreislauf 5A zugeführt werden. Die Substituatleitung 19 kann entweder an die Tropfkammer 8 stromauf der Blutkammer 3 oder an die Tropfkammer 9 stromab der Blutkammer 3 angeschlossen werden, so dass Substituat entweder der arteriellen Blutleitung 6 stromauf der Dialysierflüssigkeitskammer 3 (Prädilution) oder der venösen Blutleitung 7 stromab der Blutkammer 3 (Postdilution) zugeführt werden kann. Das Substituat wird mit einer Substituatpumpe 21 gefördert, die in der Substituatleitung 20 angeordnet ist.

Die Hämodiafiltrationsvorrichtung verfügt über eine zentrale Rechen- und Steuereinheit 22, die sämtliche für die extrakorporale Blutbehandlung erforderlichen Steuerungen der einzelnen Komponenten der Blutbehandlungsvorrichtung vornimmt. Die zentrale Rechen- und Steuereinheit 22 kann aus einen oder mehreren Komponenten bestehen. Sie kann einen oder mehrere Mikroprozessoren umfassen, die derart programmiert sind, dass die einzelnen Komponenten der Blutbehandlungsvorrichtung angesteuert werden.

Die zentrale Steuer- und Recheneinheit 22 ist über eine Steuerleitung 10' mit der Blutpumpe 10, über eine Steuerleitung 15' mit der Dialysierflüssigkeitspumpe 15, eine Steuerleitung 20' mit der Substituatpumpe 20 und eine Steuerleitung 25' mit dem Absperrorgan 25 verbunden, so dass die einzelnen Pumpen 10, 15, 21 eingeschaltet bzw. ausgeschaltet und das Absperrorgan 25 geöffnet oder geschlossen werden können und für die einzelnen Pumpen unterschiedliche Förderraten eingestellt werden können.

Darüber hinaus verfügt die Hämodiafiltrationsvorrichtung über eine nur andeutungsweise dargestellte Eingabeeinheit 23, die über eine Datenleitung 24 mit der Steuer- und Recheneinheit 22 verbunden ist. Bei der Eingabeeinheit 23 kann es sich beispielsweise um einen Touch Screen oder eine Tastatur handeln. Die Eingabeeinheit 23 kann auch eine Einheit mit einen oder mehreren Schaltern oder Reglern sein, mit denen das Personal oder der Patient selbst Einstellungen für die Blutbehandlung vornehmen kann.

Die zentrale Rechen- und Steuereinheit 22 weist Mittel 22A zur Vorgabe eines initialen Behandlungsmodus während der Anfangsphase der Behandlung auf. Diese Mittel können Teil der Maschinensteuerung sein. Beispielsweise können die Mittel ein Computerprogramm (Software) sein, das auf der Rechen- und Steuereinheit 22 läuft. Die Mittel können aber auch als Hardware ausgebildet sein.

Zu Beginn der Blutbehandlung startet die Steuer- und Recheneinheit 22 ein erstes Zeitglied, das ein erstes Zeitintervall ΔT₁ vorgibt, das beispielsweise zwischen 30 und 60 Minuten liegt. Innerhalb des ersten Zeitintervalls ΔT₁ wird die Substituatpumpe 20 bei geschlossenem Absperrorgan 25 in der Dialysierflüssigkeitszuführleitung 11 derart angesteuert, dass die Pumpe Substituat mit einer vorgegebenen Substituatrate Qₛ fördert, und die Dialysierflüssigkeitspumpe 15 Ultrafiltrat über die semipermeable Membran 2 des Dialysators 1 oder Filters aus der Dialysierflüssigkeitskammer 4 abzieht. Allein entscheidend ist, dass Dialysierflüssigkeit nicht mehr durch die Dialysierflüssigkeitskammer des Dialysators gefördert wird. Folglich wird die Hämodiafiltrationsvorrichtung während des ersten Zeitintervalls als reine Hämofiltrationsvorrichtung betrieben.

Während des ersten Zeitintervalls ΔT₁ wird die Substituatrate Qₛ vorzugsweise von Qₛ =0 auf einen Zielwert Qₛ! erhöht. Die Substituatrate Qₛ wird bei einem ersten Ausführungsbeispiel kontinuierlich mit einer gleichbleibenden Steigerungsrate von Null auf den Zielwert erhöht. Bei einer alternativen Ausführungsform wird die Substituatrate in dem ersten Zeitintervall ΔT₁ kontinuierlich mit einer zunehmenden Steigerungsrate, insbesondere exponentiell erhöht.

Da während des ersten Zeitintervalls nur ein konvektiver, nicht aber ein diffusiver Stoffaustausch stattfindet, wird die Austauschrate von Elektrolyten wie Kalium oder von Harnstoff reduziert. Dadurch wird die Gefahr des Auftretens des Dysäquilibrium-Syndroms verringert.

Nach Ablauf des ersten Zeitintervalls schalten die Mittel zur Vorgabe des initialen Behandlungsmodus von der reinen Hämofiltration auf die Hämodiafiltration um, bei der Dialysierflüssigkeit durch die zweite Kammer des Dialysators strömt und Ultrafiltrat aus der Dialysierflüssigkeitskammer abgezogen und Substituat dem extrakorporalen Blutkreislauf zugeführt werden kann Die Umschaltung erfolgt aber nicht abrupt, sondern kontinuierlich, indem nach Ablauf des ersten Zeitintervalls ein zweites Zeitintervall ΔT₂ zu laufen beginnt, das von einem zweiten Zeitglied vorgegeben wird.

Nach Ablauf des ersten Zeitintervalls wird das Absperrorgan 25 geöffnet, so dass die Blutbehandlungsvorrichtung wieder als Hämodiafiltrationsvorrichtung betrieben werden kann. Innerhalb des zweiten Zeitintervalls ΔT₂ wird die Dialysierflüssigkeitsrate Q_{d} von einem Wert Q_{d}=0 auf einen Zielwert Q_{d}! erhöht, mit dem die eigentliche Blutbehandlung nach Ablauf des zweiten Zeitintervalls durchgeführt werden soll. Dieser Zielwert kann während der Blutbehandlung konstant sein oder wiederum verändert werden. Die Erhöhung der Dialysierflüssigkeitsrate kann wieder mit einer gleichbleibenden Steigerungsrate in Form einer Rampe oder einer zunehmenden Steigerungsrate, insbesondere exponentiell erfolgen.

Nach Ablauf des zweiten Zeitintervalls ΔT₂ erfolgt die Einstellung der Flüssigkeitsraten für die eigentliche Blutbehandlung nach den Vorgaben des Arztes. Ein Eingriff in die Steuerung der Flüssigkeitsraten wird nur während der Anfangsphase ΔT=ΔT₁+ ΔT₂ der Blutbehandlung vorgenommen.

Eine bevorzugte Ausführungsform der Erfindung sieht vor, dass der Patient selbst auf der Eingabeeinheit 23 Vorgaben für das erste Zeitintervall ΔT₁ machen kann. So kann der Patient beispielsweise in Abhängigkeit von seinem momentanen Befinden das erste Zeitintervall verkürzen oder verlängern.

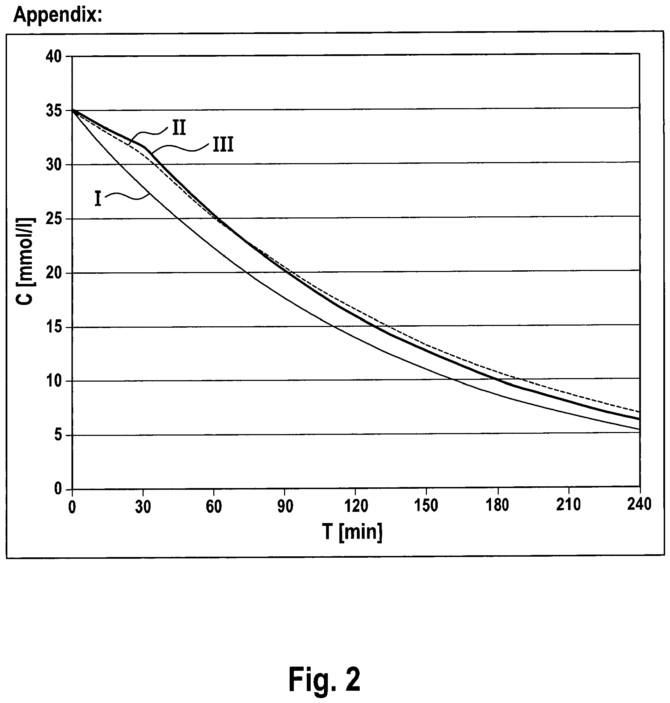

Fig. 2 zeigt die Harnstoffkonzentration C [mmol/l] als Funktion der Behandlungszeit T [min] für verschiedene Arten der Behandlung. Eine mit einer konventionellen Blutbehandlungsvorrichtung durchgeführte Hamödiafiltrationsbehandlung mit Postdilution, die einen initialen Behandlungsmodus nicht vorsieht, ist in Fig. 2 mit I, eine Hamödiafiltrationsbehandlung mit Prädilution, der ein initialer Behandlungsmodus mit einer Hämofiltration mit Prädilution vorausgeht, ist mit II und eine Hamödiafiltrationsbehandlung mit Postdilution, der ein initialer Behandlungsmodus mit einer Hämofiltration mit Postdilution vorausgeht, ist mit III bezeichnet.

Es zeigt sich, dass die Harnstoffkonzentration in der Anfangsphase der Behandlung, insbesondere in einem Zeitraum von 0 bis 60 Minuten, besonders stark abnimmt. Zum Ende der Behandlung nimmt die Harnstoffkonzentration weniger stark ab. Dies zeigt sich daran, dass die Kurve in der Anfangsphase der Behandlung steiler als in der Endphase ist. Mit der erfindungsgemäßen Vorrichtung und dem erfindungsgemäßen Verfahren wird erreicht, dass die Abnahme der Harnstoffkonzentration gerade in der Anfangsphase der Behandlung reduziert wird, indem die Blutbehandlungsvorrichtung vorzugsweise mit zunehmender Substituatrate nur als Hämofiltrationsvorrichtung betrieben wird.

Bei einer alternativen Ausführungsform der Erfindung wird in dem initialen Behandlungsmodus eine Dialysierflüssigkeitsrate von Null nicht eingestellt, d.h. die Flüssigkeitsströmung durch die Dialysierflüssigkeitskammer 2 des Dialysators wird nicht unterbrochen. Folglich braucht das Absperrorgan 25 bei dieser Ausführungsform nicht vorhanden zu sein. Es wird in dem ersten vorgegebenen Zeitintervall eine Dialysierflüssigkeitsrate eingestellt, die kleiner als die Dialysierflüssigkeitsrate in dem nachfolgenden zweiten Zeitintervall ist. In dem ersten Zeitintervall können beispielsweise Dialysierflüssigkeitsraten eingestellt werden, die unter 100 ml/min, vorzugsweise 50 ml/min, besonders bevorzugt 10/ml/min liegen. Im dem zweiten Zeitintervall kann bei der einer alternativen Ausführungsform eine Dialysierflüssigkeitsrate beispielsweise von 500 ml/min eingestellt werden. Besonders bevorzugt sind Dialysierflüssigkeitsraten größer als 270 ml/min. Die alternative Ausführungsform geht also davon aus, dass der Dialysierflüssigkeitsfluss durch die zweite Kammer des Dialysators in dem initialen Behandlungsmodus nicht zwingend vollständig unterbrochen sein muss.

## Patentansprüche

1. Vorrichtung zur extrakorporalen Blutbehandlung mit
einem extrakorporalen Blutkreislauf (5A), der eine erste Kammer (3) eines durch eine semipermeable Membran (2) in eine erste Kammer (3) und eine zweite Kammer (4) unterteilten Dialysators (1) oder Filters einschließt, und einem Flüssigkeitssystem (5B), das die zweite Kammer (4) des Dialysators (1) oder Filters einschließt,
Mitteln (10) zum Fördern von Blut im extrakorporalen Blutkreislauf (5A) durch die erste Kammer (3) des Dialysators (1) oder Filters,
Mitteln (15, 25) zum Abziehen von Ultrafiltrat aus der zweiten Kammer (4) des Dialysators (1) oder Filters sowie zum Fördern von Dialysierflüssigkeit durch die zweite Kammer (4) des Dialysators (1) oder Filters und zum Unterbrechen der Flüssigkeitsströmung in die zweite Kammer des Dialysators (1) oder Filters,
wobei die Mittel (15, 25) zum Abziehen von Ultrafiltrat sowie zum Fördern von Dialysierflüssigkeit und zum Unterbrechen der Flüssigkeitsströmung derart ausgebildet sind, dass in einem ersten Betriebsmodus Ultrafiltrat aus der zweiten Kammer (4) des Dialysators (1) oder Filters abgezogen und die Flüssigkeitsströmung in die zweite Kammer des Dialysators (1) oder Filters unterbrochen wird, und in einem zweiten Betriebsmodus Dialysierflüssigkeit durch die zweite Kammer (4) des Dialysators (1) oder Filters gefördert wird,
Mitteln (21) zum Zuführen von Substituat zu dem extrakorporalen Blutkreislauf (5A) stromauf oder stromab des Dialysators (1) oder Filters und
einer Steuer- und Recheneinheit (22), die Mittel (22A) zur Vorgabe eines initialen Behandlungsmodus nur während der Anfangsphase der Behandlung aufweist,
wobei die Mittel (22A) zur Vorgabe des initialen Behandlungsmodus derart ausgebildet sind, dass
innerhalb eines vorgegebenen ersten Zeitintervalls die Mittel (21) zum Zuführen von Substituat derart betrieben werden, dass Substituat dem extrakorporalen Blutkreislauf mit einer vorgegebenen Substituatrate Qₛ oder einem vorgegebenen Verlauf der Substituatrate Qₛ zugeführt wird,
innerhalb des vorgegebenen ersten Zeitintervalls die Mittel (15, 25) zum Abziehen von Ultrafiltrat sowie zum Fördern von Dialysierflüssigkeit und zum Unterbrechen der Flüssigkeitsströmung in dem ersten Betriebsmodus betrieben werden, so dass aus der zweiten Kammer (4) des Dialysators (1) oder Filters bei unterbrochener Flüssigkeitsströmung in die zweite Kammer des Dialysators oder Filters Ultrafiltrat abgezogen wird, und
nach Ablauf des ersten Zeitintervalls auf einen dem initialen Behandlungsmodus nachfolgenden Behandlungsmodus umgeschaltet wird, in dem die Mittel (15, 25) zum Abziehen von Ultrafiltrat sowie zum Fördern von Dialysierflüssigkeit und zum Unterbrechen der Flüssigkeitsströmung in dem zweiten Betriebsmodus betrieben werden, so dass Dialysierflüssigkeit durch die zweite Kammer (4) des Dialysators (1) oder Filters gefördert wird,
**dadurch gekennzeichnet, dass**
die Mittel (22A) zur Vorgabe des initialen Behandlungsmodus derart ausgebildet sind, dass nach Ablauf des ersten vorgegebenen Zeitintervalls die Mittel (15, 25) zum Abziehen von Ultrafiltrat sowie zum Fördern von Dialysierflüssigkeit und zum Unterbrechen der Flüssigkeitsströmung derart angesteuert werden, dass innerhalb eines zweiten vorgegebenen Zeitintervalls die Dialysierflüssigkeitsrate Q_{d} kontinuierlich mit einer gleichbleibenden Steigerungsrate oder einer zunehmenden Steigerungsrate auf einen vorgegebenen Wert erhöht wird.

2. Vorrichtung zur extrakorporalen Blutbehandlung mit
einem extrakorporalen Blutkreislauf (5A), der eine erste Kammer (3) eines durch eine semipermeable Membran (2) in eine erste Kammer (3) und eine zweite Kammer (4) unterteilten Dialysators (1) oder Filters einschließt, und einem Flüssigkeitssystem (5B), das die zweite Kammer (4) des Dialysators (1) oder Filters einschließt,
Mitteln (10) zum Fördern von Blut im extrakorporalen Blutkreislauf (5A) durch die erste Kammer (3) des Dialysators (1) oder Filters,
Mitteln (15) zum Abziehen von Ultrafiltrat aus der zweiten Kammer (4) des Dialysators (1) oder Filters sowie zum Fördern von Dialysierflüssigkeit durch die zweite Kammer (4) des Dialysators (1) oder Filters,
Mitteln (21) zum Zuführen von Substituat zu dem extrakorporalen Blutkreislauf (5A) stromauf oder stromab des Dialysators (1) oder Filters und
einer Steuer- und Recheneinheit (22), die Mittel (22A) zur Vorgabe eines initialen Behandlungsmodus nur während der Anfangsphase der Behandlung aufweist,
wobei die Mittel (22A) zur Vorgabe des initialen Behandlungsmodus derart ausgebildet sind, dass
innerhalb eines vorgegebenen ersten Zeitintervalls die Mittel (15) zum Abziehen von Ultrafiltrat sowie zum Fördern von Dialysierflüssigkeit derart betrieben werden, dass aus der zweiten Kammer (4) des Dialysators (1) Dialysierflüssigkeit mit einer ersten Dialysierflüssigkeitsrate gefördert wird, und nach Ablauf des ersten Zeitintervalls auf einen dem initialen Behandlungsmodus nachfolgenden Behandlungsmodus umgeschaltet wird, in dem die Mittel (15) zum Abziehen von Ultrafiltrat sowie zum Fördern von Dialysierflüssigkeit derart betrieben werden,
dass Dialysierflüssigkeit durch die zweite Kammer (4) des Dialysators (1) oder Filters mit einer zweiten Dialysierflüssigkeitsrate gefördert wird, wobei die erste Dialysierflüssigkeitsrate kleiner als die zweite Dialysierflüssigkeitsrate ist,
**dadurch gekennzeichnet, dass**
die Mittel (22A) zur Vorgabe des initialen Behandlungsmodus derart ausgebildet sind, dass
nach Ablauf des ersten vorgegebenen Zeitintervalls die Mittel (15) zum Abziehen von Ultrafiltrat sowie zum Fördern von Dialysierflüssigkeit derart angesteuert werden, dass innerhalb eines zweiten vorgegebenen Zeitintervalls die Dialysierflüssigkeitsrate Q_{d} kontinuierlich mit einer gleichbleibenden Steigerungsrate oder einer zunehmenden Steigerungsrate auf einen vorgegebenen Wert erhöht wird,
innerhalb des vorgegebenen ersten Zeitintervalls die Mittel (21) zum Zuführen von Substituat derart betrieben werden, dass Substituat dem extrakorporalen Blutkreislauf mit einer vorgegebenen Substituatrate Qₛ oder einem vorgegebenen Verlauf der Substituatrate Qₛ zugeführt wird, und
innerhalb des vorgegebenen ersten Zeitintervalls die Mittel (15) zum Abziehen von Ultrafiltrat sowie zum Fördern von Dialysierflüssigkeit derart betrieben werden, dass aus der zweiten Kammer (4) des Dialysators (1) oder Filters Ultrafiltrat abgezogen wird, wobei Dialysierflüssigkeit mit der ersten Dialysierflüssigkeitsrate gefördert wird.

3. Vorrichtung nach Anspruch 1 oder 2, **dadurch gekennzeichnet, dass** die Mittel (22A) zur Vorgabe des initialen Behandlungsmodus derart ausgebildet sind, dass die Mittel (21) zum Zuführen von Substituat innerhalb des vorgegebenen ersten Zeitintervalls derart angesteuert werden, dass die Substituatrate Qₛ innerhalb des ersten Zeitintervalls erhöht wird.

4. Vorrichtung nach Anspruch 3, **dadurch gekennzeichnet, dass** die Mittel (21) zum Zuführen von Substituat derart angesteuert werden, dass die Substituatrate Qₛ innerhalb des vorgegebenen ersten Zeitintervalls kontinuierlich mit einer gleichbleibenden Steigerungsrate erhöht wird.

5. Vorrichtung nach Anspruch 3, **dadurch gekennzeichnet, dass** die Mittel (21) zum Zuführen von Substituat derart angesteuert werden, dass die Substituatrate Qₛ innerhalb des vorgegebenen ersten Zeitintervalls kontinuierlich mit einer zunehmenden Steigerungsrate erhöht wird.

6. Vorrichtung nach einem der Ansprüche 1 bis 5, **dadurch gekennzeichnet, dass** die Blutbehandlungsvorrichtung eine Eingabeeinheit (23) zur Eingabe der Länge und/oder zur Veränderung der Länge des ersten Zeitintervalls aufweist.

7. Vorrichtung nach Anspruch 1 oder einem der Ansprüche 3 bis 6, **dadurch gekennzeichnet, dass** der extrakorporale Blutkreislauf (5A) eine zu der ersten Kammer (3) des Dialysators (1) oder Filters führende Blutzuführleitung (6) und von der ersten Kammer (3) des Dialysators (1) oder Filters abgehende Blutabführleitung (7) aufweist, und das Flüssigkeitssystem (5B) eine zu der zweiten Kammer des Dialysators oder Filters führende Dialysierflüssigkeitszuführleitung (11) und eine von der zweiten Kammer des Dialysators oder Filters abgehende Dialysierflüssigkeitsabführleitung (12) aufweist, wobei die Mittel (15, 25) zum Fördern von Dialysierflüssigkeit und Abziehen von Ultrafiltrat ein in der Dialysierflüssigkeitszuführleitung angeordnetes Absperrorgan (25) und eine in der Dialysierflüssigkeitsabführleitung (12) angeordnete Dialysierflüssigkeitspumpe (15) aufweisen.

8. Vorrichtung nach einem der Ansprüche 1 bis 7, **dadurch gekennzeichnet, dass** das Flüssigkeitssystem (5B) eine zu der Blutzuführleitung (6) oder der Blutabführleitung (7) führende Substituatleitung (20) aufweist, wobei die Mittel (21) zum Zuführen von Substituat eine in der Substituatleitung (20) angeordnete Substituatpumpe (21) aufweisen.

## Claims

1. Apparatus for extra-corporeal blood treatment comprising
an extra-corporeal blood circuit (5A) which includes a first chamber (3) of a dialyser (1) or filter which is divided into a first chamber (3) and a second chamber (4) by a semi-permeable membrane (2), and a fluid system (5B) which includes the second chamber (4) of the dialyser (1) or filter,
means (10) for feeding blood through the first chamber (3) of the dialyser (1) or filter in the extra-corporeal blood circuit (5A),
means (15, 25) for withdrawing ultrafiltrate from the second chamber (4) of the dialyser (1) or filter and for feeding dialysis fluid through the second chamber (4) of the dialyser (1) or filter and for interrupting the fluid flow into the second chamber of the dialyser (1) or filter,
wherein the means (15, 25) for withdrawing ultrafiltrate and for feeding dialysis fluid and for interrupting the fluid flow are configured such that ultrafiltrate is withdrawn from the second chamber (4) of the dialyser (1) or filter in a first operating mode and the fluid flow into the second chamber of the dialyser (1) or filter is interrupted and, in a second operating mode, dialysis fluid is fed through the second chamber (4) of the dialyser (1) or filter,
means (21) for feeding substituate into the extra-corporeal blood circuit (5A) upstream or downstream of the dialyser (1) or filter, and
a control and computing unit (22) comprising means (22A) for presetting an initial mode of treatment only during the opening phase of the treatment,
the means (22A) for presetting the initial mode of treatment being configured such that,
within a preset first interval of time, the means (21) for feeding substituate are operated in such a way that substituate is fed to the extra-corporeal blood circuit at a preset substituate flow rate Qₛ or a preset progression of the substituate rate Qₛ,
the means (15, 25) for withdrawing ultrafiltrate and for feeding dialysis fluid and for interrupting the fluid flow being operated in the first mode of operation over the preset first interval of time, ultrafiltrate thus being withdrawn from the second chamber (4) of the dialyser (1) or filter when the flow of fluid is interrupted into the second chamber of the dialyser or filter and,
on expiry of the first interval of time, a switch is made to a mode of treatment which follows on from the initial mode of treatment and in which the means (15, 25) for withdrawing ultrafiltrate and for feeding dialysis fluid and for interrupting the fluid flow are operated in the second mode of operation, dialysis fluid thus being fed through the second chamber (4) of the dialyser (1) or filter.
**characterised in that**
the means (22A) for presetting the initial mode of treatment are configured such that, on expiry of the preset first interval of time, the means (15, 25) for withdrawing ultrafiltrate and for feeding dialysis fluid and for interrupting the fluid flow are operated in such a way that, over a second preset interval of time, the dialysis fluid flow rate Q_{d} is increased continuously at a constant rate of increase or an increasing rate to a preset value.

2. Apparatus for extracorporeal blood treatment comprising
an extra-corporeal blood circuit (5A) which includes a first chamber (3) of a dialyser (1) or filter which is divided into a first chamber (3) and a second chamber (4) by a semi-permeable membrane (2), and a fluid system (5B) which includes the second chamber (4) of the dialyser (1) or filter,
means (10) for feeding blood through the first chamber (3) of the dialyser (1) or filter in the extra-corporeal blood circuit (5A),
means (15) for withdrawing ultrafiltrate from the second chamber (4) of the dialyser (1) or filter and for feeding dialysis fluid through the second chamber (4) of the dialyser (1) or filter,
means (21) for feeding substituate to the extracorporeal blood circuit (5A) upstream or downstream of the dialyser (1) or filter, and
a control and computing unit (22) comprising means (22A) for presetting an initial mode of treatment only during the opening phase of the treatment,
wherein the means (15, 25) for presetting the initial mode of treatment are configured such that
the means (15) for withdrawing ultrafiltrate and for feeding dialysis fluid are operated over a preset first interval of time such that dialysis fluid is fed from the second chamber (4) of the dialyser (1) or filter at a first dialysis fluid rate, and after the first time interval has elapsed, the switch is made to an operating mode following the initial treatment mode, in which the means (15) for withdrawing ultrafiltrate and for feeding dialysis are operated such that dialysis fluid is fed through the second chamber (4) of the dialyser (1) or filter at a second dialysis fluid rate, wherein the first dialysis fluid rate is smaller than the second dialysis fluid rate,
**characterised in that**
the means (22A) for presetting the initial mode of treatment are configured such that,
on expiry of the preset first interval of time, the means (15) for withdrawing ultrafiltrate and for feeding dialysis are operated in such a way that, over a second preset interval of time, the dialysis fluid flow rate Q_{d} is continuously increased at a constant rate of increase or an increasing rate to a preset value,
within the preset first interval of time, the means (21) for feeding substituate are operated in such a way that substituate is fed to the extra-corporeal blood circuit at a preset substituate flow rate Qₛ or a preset progression of the substituate rate Qₛ, and
within the preset first interval of time, the means (15) for withdrawing ultrafiltrate and for feeding dialysis are operated, ultrafiltrate thus being withdrawn from the second chamber (4) of the dialyser (1) or filter and dialysis fluid is fed at the first dialysis fluid rate.

3. Apparatus according to claim 1 or 2, **characterised in that** the means (22A) for presetting the initial treatment mode are configured such that the means (21) for feeding substituate within the preset first time interval are operated in such a way that the substituent rate Qₛ is increased within the first time interval.

4. Apparatus according to claim 3, **characterised in that** the means (21) for feeding substituate are operated in such a way that the substituate flow rate Qₛ is increased continuously over the preset first interval of time at a constant rate of increase.

5. Apparatus according to claim 3, **characterised in that** the means (21) for feeding substituate are operated in such a way that the substituate flow rate Qₛ is increased continuously over the preset first interval of time at an increasing rate of increase.

6. Apparatus according to one of claims 1 to 5, **characterised in that** the blood treatment apparatus has an input unit (23) for the input of the length of the first interval of time and/or for changing its length.

7. Apparatus according to claim 1 or one of the claims 3 to 6, **characterised in that** the extra-corporeal blood circuit (5A) has a blood infeed line (6) which runs to the first chamber (3) of the dialyser (1) or filter, and a blood outfeed line (7) which leads from the first chamber (3) of the dialyser (1) or filter, and the fluid system (5B) has a dialysis-fluid infeed line (11) which runs to the second chamber of the dialyser or filter, and a dialysis-fluid outfeed line (12) which leads from the second chamber of the dialyser or filter, the means (15, 25) for feeding dialysis fluid and withdrawing ultrafiltrate having a shut-off member (25) arranged in the dialysis-fluid infeed line and a dialysis-fluid pump (15) arranged in the dialysis-fluid outfeed line (12).

8. Apparatus according to one of claims 1 to 7, **characterised in that** the fluid system (5B) has a substituate line (20) running to the blood infeed line (6) or blood outfeed line (7), the means (21) for feeding substituate having a substituate pump (21) arranged in the substituate line (20).

## Revendications

1. Dispositif pour le traitement extracorporel du sang comportant
une circulation sanguine extracorporelle (5A) qui renferme une première chambre (3) d'un dialyseur (1) ou filtre subdivisée par une membrane semi-perméable (2) en une première chambre (3) et une deuxième chambre (4), et un système de fluide (5B) qui renferme la deuxième chambre (4) du dialyseur (1) ou filtre,
des moyens (10) pour acheminer du sang dans la circulation sanguine extracorporelle (5A) au travers de la première chambre (3) du dialyseur (1) ou filtre,
des moyens (15, 25) pour retirer un ultrafiltrat de la deuxième chambre (4) du dialyseur (1) ou filtre et pour acheminer du fluide de dialyseur au travers de la deuxième chambre (4) du dialyseur (1) ou filtre et pour interrompre le courant de fluide dans la deuxième chambre du dialyseur (1) ou filtre,
dans lequel les moyens (15, 25) pour retirer un ultrafiltrat ainsi que pour acheminer le fluide de dialyseur et pour interrompre le courant de fluide sont formés de telle sorte que dans un premier mode de fonctionnement, de l'ultrafiltrat est retiré de la deuxième chambre (4) du dialyseur (1) ou filtre et le courant de fluide est interrompu dans la deuxième chambre du dialyseur (1) ou filtre, et dans un deuxième mode de fonctionnement, du fluide de dialyseur est acheminé au travers de la deuxième chambre (4) du dialyseur (1) ou filtre,
des moyens (21) pour acheminer du substitut à la circulation sanguine extracorporelle (5A) en amont ou en aval du dialyseur (1) ou filtre et à une unité de commande et de calcul (22) qui présente des moyens (22A) pour prédéterminer un mode de traitement initial uniquement pendant la phase de début du traitement,
dans lequel les moyens (22A) pour prédéterminer le mode de traitement initial sont formés de telle sorte que
à l'intérieur d'un premier intervalle temporel prédéterminé, les moyens (21) pour acheminer un substitut sont actionnés de telle sorte que du substitut est acheminé à la circulation sanguine extracorporelle avec un taux de substitut Qₛ prédéterminé ou une évolution prédéterminée du taux de substitut Qₛ,
à l'intérieur du premier intervalle temporel prédéterminé, les moyens (15, 25) pour retirer de l'ultrafiltrat ainsi que pour acheminer du fluide de dialyseur et pour interrompre le courant de fluide dans le premier mode de fonctionnement sont actionnés, de sorte que de l'ultrafiltrat soit retiré de la deuxième chambre (4) du dialyseur (1) ou filtre lorsque le courant de fluide est interrompu dans la deuxième chambre du dialyseur ou filtre, et
après l'écoulement du premier intervalle temporel, on passe à un mode de traitement suivant le mode de traitement initial, dans lequel les moyens (15, 25) pour retirer de l'ultrafiltrat ainsi que pour acheminer du fluide de dialyseur et pour interrompre le courant de fluide sont actionnés dans le deuxième mode de fonctionnement, de sorte que du fluide de dialyseur soit acheminé au travers de la deuxième chambre (4) du dialyseur (1) ou filtre, **caractérisé en ce que**
les moyens (22A) pour prédéterminer le mode de traitement initial sont formés de telle sorte qu'après l'écoulement du premier intervalle temporel prédéterminé, les moyens (15, 25) pour retirer l'ultrafiltrat ainsi que pour acheminer du fluide de dialyseur et pour interrompre le courant de fluide sont commandés de sorte qu'à l'intérieur d'un deuxième intervalle temporel prédéterminé, le taux de fluide de dialyseur Q_{d} soit augmenté en continu à une valeur prédéterminée alors que le taux d'augmentation reste identique ou que le taux d'augmentation s'accroît.

2. Dispositif de traitement extracorporel du sang, comportant
une circulation sanguine extracorporelle (5A) qui renferme une première chambre (3) d'un dialyseur (1) ou filtre subdivisée par une membrane semi-perméable (2) en une première chambre (3) et une deuxième chambre (4), et un système de fluide (5B) qui renferme la deuxième chambre (4) du dialyseur (1) ou filtre,
des moyens (10) pour acheminer du sang dans la circulation sanguine extracorporelle (5A) au travers de la première chambre (3) du dialyseur (1) ou filtre,
des moyens (15) pour retirer un ultrafiltrat de la deuxième chambre (4) du dialyseur (1) ou filtre et pour acheminer du fluide de dialyseur au travers de la deuxième chambre (4) du dialyseur (1) ou filtre,
des moyens (21) pour acheminer du substitut à la circulation sanguine extracorporelle (5A) en amont ou en aval du dialyseur (1) ou filtre et
une unité de commande et de calcul (22) qui présente des moyens (22A) pour prédéterminer un mode de traitement initial uniquement pendant la phase de début du traitement,
dans lequel les moyens (22A) pour prédéterminer le mode de traitement initial sont formés de telle sorte que
à l'intérieur d'un premier intervalle temporel prédéterminé, les moyens (15) pour retirer de l'ultrafiltrat ainsi que pour acheminer du fluide de dialyseur sont actionnés, de sorte que du fluide de dialyseur de la deuxième chambre (4) du dialyseur (1) soit acheminé avec un premier taux de fluide de dialyseur, et après l'écoulement du premier intervalle temporel, on passe à un mode de traitement suivant le mode de traitement initial, dans lequel les moyens (15) pour retirer de l'ultrafiltrat ainsi que pour acheminer du fluide de dialyseur sont actionnés de sorte que du fluide de dialyseur soit acheminé au travers de la deuxième chambre (4) du dialyseur (1) ou filtre avec un deuxième taux de fluide de dialyseur, dans lequel le premier taux de fluide de dialyseur est inférieur au deuxième taux de fluide de dialyseur,
**caractérisé en ce que**
les moyens (22A) pour prédéterminer le mode de traitement initial sont formés de telle sorte que
après l'écoulement du premier intervalle temporel prédéterminé, les moyens (15) pour retirer l'ultrafiltrat ainsi que pour acheminer du fluide de dialyseur sont commandés de sorte qu'à l'intérieur d'un deuxième intervalle temporel prédéterminé, le taux de fluide de dialyseur Q_{d} soit augmenté en continu à une valeur prédéterminée alors que le taux d'augmentation reste identique ou que le taux d'augmentation s'accroît,
à l'intérieur d'un premier intervalle temporel prédéterminé, les moyens (21) pour acheminer un substitut sont actionnés de sorte que du substitut soit acheminé à la circulation sanguine extracorporelle avec un taux de substitut Qₛ prédéterminé ou une évolution prédéterminée du taux de substitut Qₛ, et
à l'intérieur du premier intervalle temporel prédéterminé, les moyens (15) pour retirer de l'ultrafiltrat ainsi que pour acheminer du fluide de dialyseur sont actionnés, de sorte que de l'ultrafiltrat soit retiré de la deuxième chambre (4) du dialyseur (1) ou filtre, dans lequel le fluide de dialyseur est acheminé avec le premier taux de fluide de dialyseur.

3. Dispositif selon la revendication 1 ou 2, **caractérisé en ce que** les moyens (22A) pour prédéterminer le mode de traitement initial sont formés de telle sorte que les moyens (21) pour acheminer du substitut à l'intérieur du premier intervalle temporel prédéterminé sont commandés de sorte que le taux de substitut Qₛ soit augmenté à l'intérieur du premier intervalle temporel.

4. Dispositif selon la revendication 3, **caractérisé en ce que** les moyens (21) pour acheminer du substitut sont commandés de sorte que le taux de substitut Qₛ soit augmenté en continu à l'intérieur du premier intervalle temporel prédéterminé alors que le taux d'augmentation reste identique.

5. Dispositif selon la revendication 3, **caractérisé en ce que** les moyens (21) pour acheminer du substitut sont commandés de sorte que le taux de substitut Qₛ soit augmenté en continu à l'intérieur du premier intervalle temporel prédéterminé alors que le taux d'augmentation s'accroît.

6. Dispositif selon l'une des revendications 1 à 5, **caractérisé en ce que** le dispositif de traitement du sang présente une unité d'entrée (23) pour entrer la longueur et/ou pour modifier la longueur du premier intervalle temporel.

7. Dispositif selon la revendication 1 ou l'une des revendications 3 à 6, **caractérisé en ce que** la circulation sanguine extracorporelle (5A) présente une conduite d'acheminement de sang (6) conduisant à la première chambre (3) du dialyseur (1) ou filtre et une conduite d'évacuation (7) de sang partant de la première chambre (3) du dialyseur (1) ou filtre, et le système de fluide (5B) présente une conduite d'acheminement de fluide de dialyseur (11) conduisant à la deuxième chambre du dialyseur ou filtre et une conduite d'évacuation de fluide de dialyseur (12) partant de la deuxième chambre du dialyseur ou filtre, dans lequel les moyens (15, 25) pour acheminer du fluide de dialyseur et retirer de l'ultrafiltrat présentent un organe de blocage (25) disposé dans la conduite d'acheminement de fluide de dialyseur et une pompe de fluide de dialyseur (15) disposée dans la conduite d'évacuation du fluide de dialyseur (12).

8. Dispositif selon l'une des revendications 1 à 7, **caractérisé en ce que** le système de fluide (5B) présente une conduite de substitut (20) conduisant à la conduite d'acheminement de sang (6) ou la conduite d'évacuation de sang (7), dans lequel les moyens (21) pour acheminer du substitut présentent une pompe de substitut (21) disposée dans la conduite de substitut (20).
